# EUROPEAN PATENT SPECIFICATION

(11) **EP 1 675 531 B1**
(45) Date of publication and mention of the grant of the patent: **31.12.2008**
(21) Application number: 03759675.6
(22) Date of filing: 02.10.2003
(51) Int. Cl.: A61F 2/42

(54) **ANKLE JOINT PROSTHESIS**
SPRUNGGELENKSPROTHESE
PROTHESE DE L'ARTICULATION DE LA CHEVILLE

(43) Date of publication of application: 05.07.2006
(73) Proprietor: Concepts in Medicine III, LLC, West Friendship, MD 21794 (US)
(72) Inventor: SCHON, Lew, C., Baltimore, MD 21208 (US); CHIODO, Christopher, Walpole, MA 02081 (US); PARKS, Brent, G., West Friendship, MD 21794 (US); HERBST, Steven, Selma, IN 47383 (US); LAU, Johnny, Toronto, Ontario M4Y2W4 (CA)
(74) Representative: Bugnion Genève
(86) International application number: PCT/US2003/031257
(87) International publication number: WO 2005/041823

(56) References cited:
- EP-A- 0 800 803
- DE-U- 8 812 806
- FR-A- 2 676 917
- US-A- 4 470 158
- US-A- 5 326 365

## Description

### BACKGROUND OF THE INVENTION

### 1. FIELD OF THE INVENTION

This invention relates to ankle prostheses. The document FR-A-2676917 represents the closest prior art.

### 2. DESCRIPTION OF PRIOR ART

For many years there has been considerable interest and activity with respect to ankle joint replacements, in which the degenerative articular surfaces are removed and replaced with an artificial joint called a prosthesis, as a viable approach to the treatment of diseased or injured ankle joints.

Fusion has long been an alternative to ankle arthroplasty. This approach has its drawbacks. For example, there is a loss of motion in the ankle joint which may cause difficulties with other associated parts of the foot and leg.

Many types of ankle joint prostheses have been developed over the past thirty years. After initial encouraging results, the reputation of ankle arthroplasty was somewhat damaged based on long-term, follow-up clinical studies which revealed the frequent failures of such implants due mainly to the inadequate restoration of the original mobility and the poor stability of the resulting ankle complex. Problems which many have speculated are due to our poor understanding of the relative contribution of the ligamentous structures and articular surfaces in providing passive and active stability for the ankle joint.

The ongoing problems with ankle arthrodesis have encouraged numerous ankle arthroplasty designs. The early designs all feature two-component prostheses having talar and tibial components for respective attachment to the talar and tibia bones of the ankle. For example, see U.S. Patent Nos. 4,156,944, 4,069,518, 4,021,864, 3,987,500, 3,975,778, 3,896,503, 3,896, 502, 3,889,300, 3,886,599, 3,872, 519, and 3,839,742.

Despite the multitude of these designs, none of them yielded clinical results comparable to those achieved with total hip and knee replacement surgeries. Aseptic loosening of the tibial and/or talar components is reportedly the most frequent cause of failure, but complications also included deep infections, dehiscence of the surgical wound, lateral and/or medial subluxation of the floating meniscus and lateral talofibular joint impingement.

The most recent prosthesis design feature three components and include a floating, intermediate element that has been introduced to allow full congruence at the articular surfaces in all joint positions in order to minimize wear of the components while coping with the multi-axial nature of the axial rotation of the ankle. These designs all feature a planar and a curved surface for the intermediate element in order to allow a controlled freedom of motion relative to the tibial component, allowing controlled anterior-posterior as well as medial-lateral motion in such a way as to reduce wear of the surfaces and the stress at the interface between the bone and the tibial component of the prosthesis. For example, see U.S. Patent Nos. 4,470,158, and 5,766,259. These three component designs are also reported to have exhibited problems with aseptic loosening of the tibial and/or talar components, migration of the prosthesis and inadequate motion in the replaced joint.

A common characteristic among these previous total ankle prostheses is that they are inserted through incisions made with an anterior approach to the ankle joint. This approach requires making large incisions at the ankle and moving the tendons and other soft tissue aside; thus violating important anterior soft-tissue structures and, more importantly, neurovascular structures that provide blood flow to the talus.

Despite the extensive development of ankle joint prostheses, they often continue to exhibit less than desirable performance. Thus, there exists a continuing need for the development of new and improved types of such devices. There is also a need for a less invasive surgical method to install such a prostheses so as to yield improved healing and a decrease in the failure rate of such devices.

### 3. OBJECTS AND ADVANTAGES

There has been summarized above, rather broadly, the prior art that is related to the present invention in order that the context of the present invention may be better understood and appreciated. In this regard, it is instructive to also consider the objects and advantages of the present invention.

It is an object of the present invention to provide an improved ankle joint prosthesis that decreases the failure rate currently being experienced by such devices.

It is an object of the present invention to provide an ankle joint prosthesis having components that have crescentic shaped surfaces for attachment with the adjoining tibia and talus bones so as to provide more surface area for bony ingrowth or cement fixation than that provided by standard prostheses which use flat surfaces.

It is an object of the present invention to present an ankle joint prosthesis with different levels of constraint, ranging from unconstrained to semi-constrained. This provides options for dealing with different clinical situations. Ultimately, the goal will be to use an ankle joint prosthesis, which minimizes wear and enhances the longevity of the implant.

It is a still further object of the present invention to provide a device that will advance the effectiveness of ankle joint replacements in orthopedic medicine.

These and other objects and advantages of the present invention will become readily apparent as the invention is better understood by reference to the accompanying summary, drawings and the detailed description that follows.

### SUMMARY OF THE INVENTION

Recognizing the need for the development of improved ankle joint prostheses, the present invention is generally directed to satisfying the needs set forth above and overcoming the disadvantages identified with prior art devices.

In accordance with the present invention, the foregoing need can be satisfied by providing an ankle joint prosthesis adapted to involve the patient's distal tibia and talus. This prosthesis may have many embodiments.

In a preferred, mobile bearing embodiment, the prosthesis comprises tibial, talar and mobile bearing components that are laterally to medially implanted in the patient. The tibial component's superior (top) surface has convex curvature in the anterior to posterior direction and is configured so as to approximate and match with the curvature of a prepared portion of the distal tibia; its inferior (bottom) surface is approximately flat. The talar component's top surface has one of two forms of curvature. It has only convex curvature in the anterior to posterior direction or it has this curvature plus concave curvature in the lateral to medial direction. Its bottom surface has one of two shaped surfaces. It has concave curvature in the anterior to posterior direction and is configured so as to approximate and match with the curvature of a prepared portion of the talus or it has an approximately flat surface. The mobile bearing component's top surface is approximately flat, and its bottom surface has curvature that is complementary to the curvature of the talar component's top surface.

In a preferred, semi-constrained bearing embodiment, the prosthesis comprises tibial, semi-constrained bearing and talar components that are laterally to medially implanted in the patient. In this instance, the tibial component's top surface has convex curvature in the anterior to posterior direction and is configured so as to approximate and match with the curvature of a prepared portion of the distal tibia; its bottom surface has concave curvature in the anterior to posterior direction. The talar component's top surface has curvature that is configured to be complimentary with the curvature of the semi-constrained bearing component's bottom surface so as to allow maximal surface contact. Its bottom surface has concave curvature in the anterior to posterior direction and has protrusions at its anterior and posterior ends that protrude downward or it has a flat surface in the anterior to posterior direction, with or without protrusions at the anterior and posterior ends that protrude downward. The semi-constrained bearing component has a top surface with curvature that is complimentary with the curvature found in the bottom surface of the tibial component so as to lock these .. surfaces together in various levels of constraint. Polyethylene or another suitable bearing material is used for constructing the semi-constrained bearing component.

Thus, there has been summarized above, rather broadly, the present invention in order that the detailed description that follows may be better understood and appreciated. There are, of course, additional features of the invention that will be described hereinafter and which will form the subject matter of any eventual claims to this invention.

### BRIEF DESCRIPTION OF THE DRAWINGS

FIG. 1 is a lateral elevational view of a right, human foot.
FIG. 2 is a similar view of the foot in FIG. 1 and showing the fibula having been cut and moved down to gain lateral access to the ankle joint.
FIG. 3 is a similar view of the foot in FIG. 1 and showing the ankle joint distracted so as to provide better lateral access to the ankle joint.
FIG. 4 is a similar view of the foot in FIG. 1 and showing by dashed lines the shape of the cuts to be made in the surfaces of the distal tibia and talar dome.
FIG. 5 is a perspective, side view of a special cutting guide that is used to make the cuts in the surfaces of the distal tibia and talar dome.
FIG. 6 shows the special cutting guide of FIG. 5 in use for surgically removing a portion of the adjoining tissue between the top of the talus and the bottom of the tibia.
FIG. 7 shows an anterior view of the alignment rod being used with the special cutting guide.
FIG. 8 shows an lateral view of the alignment rod being used with the special cutting guide.
FIG. 9 is a similar view of the foot in FIG. 1 and showing the distal tibia and talar dome after the necessary portions have been removed.
FIG. 10A is an anterior, in-situ view of a talar, lateral surface cutting guide that is used to cut the lateral surface of the talus dome.
FIG. 10B is a close-up view of the cutting guide shown in FIG. 10A.
FIG.11A is a lateral, in-situ view of the cutting guide shown in FIG. 10A.
FIG. 11B is a close-up view of the cutting guide shown in FIG. 11A.
FIG. 12A is a perspective view of a recess cutting guide that is used to make the recesses in the surfaces of the talar dome and the distal tibia.
FIG. 12B is a lateral view of the recess cutting guide shown in FIG. 12A.
FIG. 12C is a medial view of the recess cutting guide shown in FIG. 12A.
FIG. 13A is an anterior, in-situ view of the recess cutting guide shown in FIG. 12.
FIG. 13B is a close-up view of the recess cutting guide shown in FIG. 13A.
FIG. 14A is a lateral, in-situ view of the recess cutting guide shown in FIG. 12.
FIG. 14B is a close-up view of the recess cutting guide shown in FIG. 14A.
FIG. 15 is a perspective, medial side view of a preferred, mobile bearing embodiment of the ankle joint prosthesis of the present invention.
FIG. 16 is a similar view of the foot in FIG. 1 and showing the mobile bearing, ankle joint prosthesis that is to be inserted in place of the removed portions of the distal tibia and talar dome.

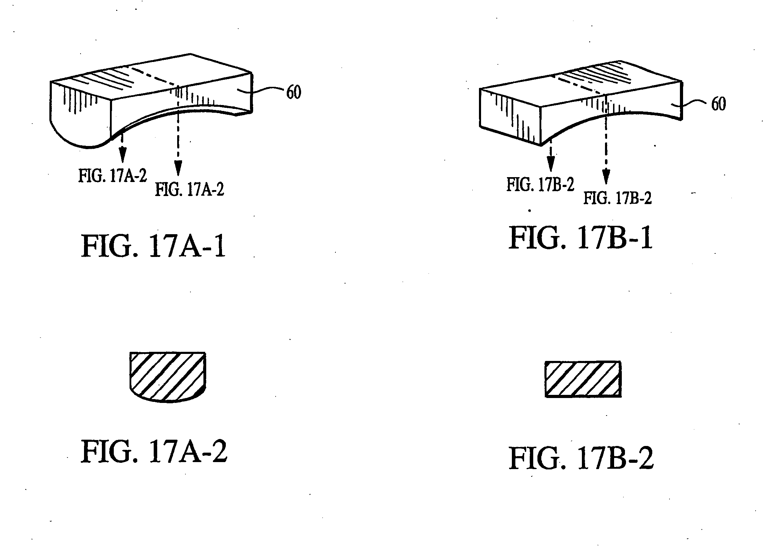

FIGS. 17A-1 and 17A-2 show perspective and cross-sectional views of a preferred embodiment of the mobile bearing component of the present invention in which the component's bottom surface is saddle shaped and has concave curvature in the anterior to posterior direction and convex curvature in the lateral to posterior direction.
FIGS. 17B-1 and 17B-2 show perspective and cross-sectional views of a preferred embodiment of the mobile bearing component of the present invention in which the component's bottom surface has concave curvature only in the anterior to posterior direction.

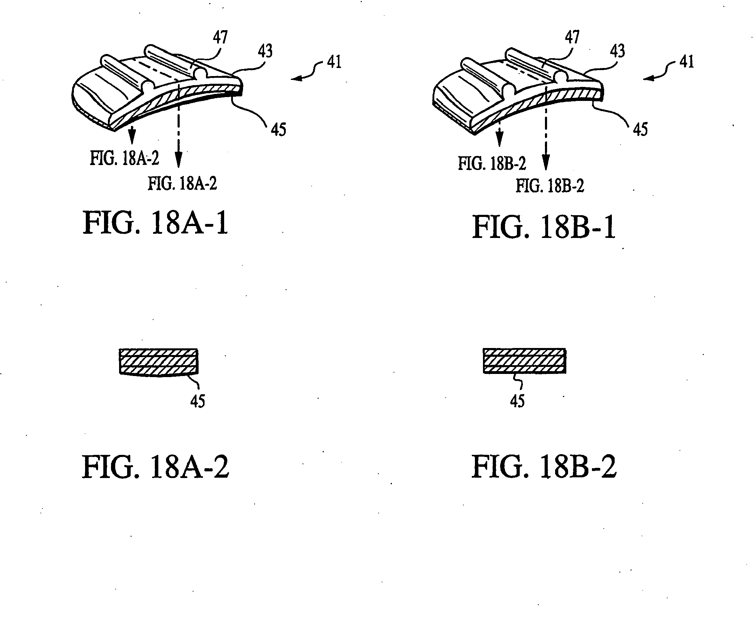

FIGS. 18A-1 and 18-2 show perspective and a cross-sectional views of a preferred embodiment of the tibial component in a semi-constrained version of the present invention in which the component's bottom surface is saddle shaped and has concave curvature in the anterior to posterior direction and convex curvature in the lateral to posterior direction.
FIGS. 18B-1 and 18B-2 show perspective and a cross-sectional views of a preferred embodiment of the tibial component in a semi-constrained version of the present invention in which the component's bottom surface has concave curvature only in the anterior to posterior direction.

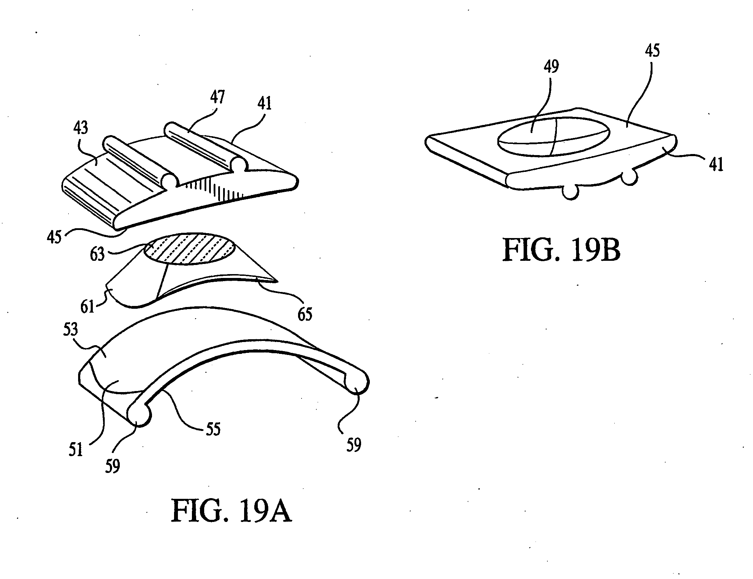

FIG. 19A is a perspective, medial side view of a semi-constrained bearing embodiment in which the bearing component's top surface has a dome that partially locks this component into the matching concavity or recess that exists in the tibial component's bottom surface.
FIG. 19B shows the bottom view of the tibial component shown in FIG. 19A.

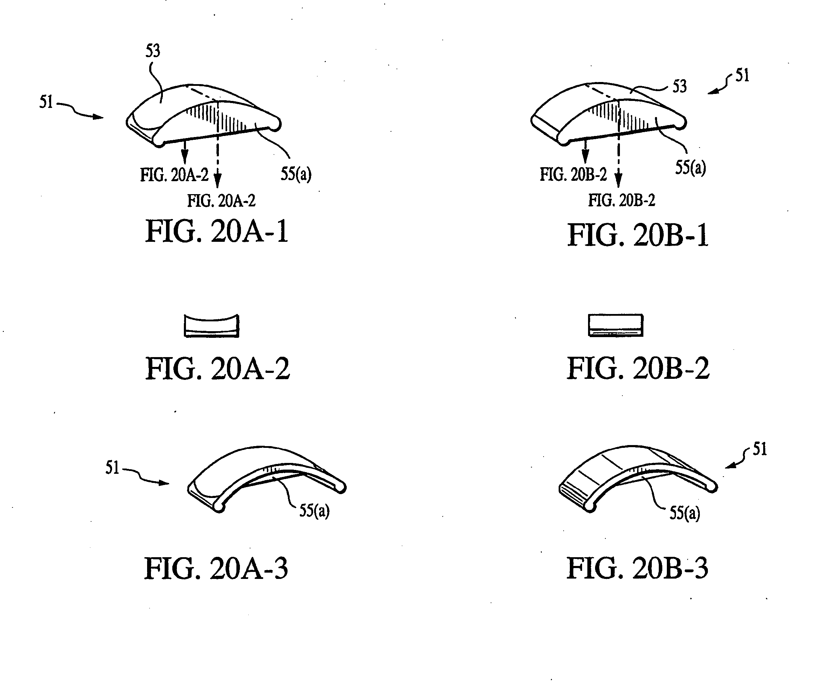

FIGS. 20A-1 to 20A-3 show respective lateral, cross-sectional and medial views of a preferred embodiment of the talar components used with the semi-constrained version of the prosthesis of the present invention.
FIGS. 20B-1 to 20B-3 show respective lateral, cross-sectional and medial views of a preferred embodiment of the talar components used with the semi-constrained version of the prosthesis of the present invention.

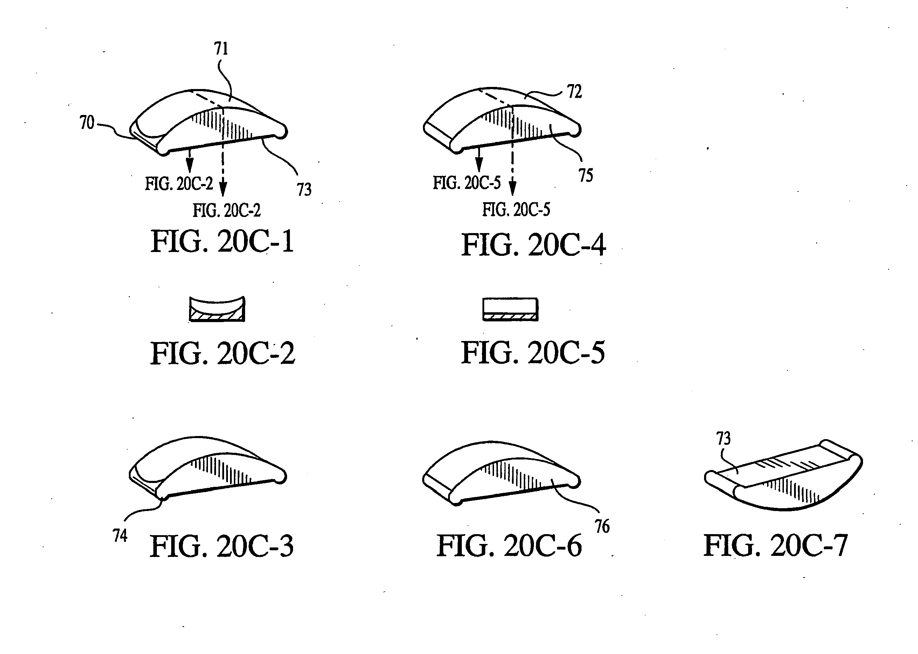

FIGS. 20C-1 to 20C-7 show respective lateral, cross-sectional, medial, and bottom views of a preferred embodiment of the talar components used with either the mobile-bearing or the semi-constrained version of the prosthesis of the present invention when a flat cut is required on the talus.

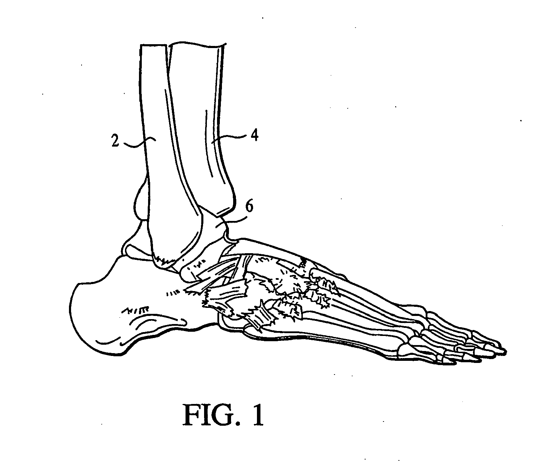

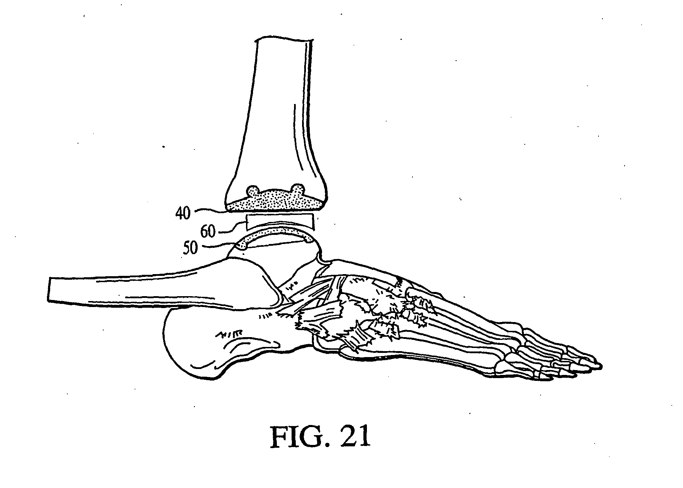

FIG. 21 is a similar view of the foot in FIG. 1 and showing the mobile bearing, ankle joint prosthesis components after they have been inserted in place of the removed portions of the distal tibia and talar dome.

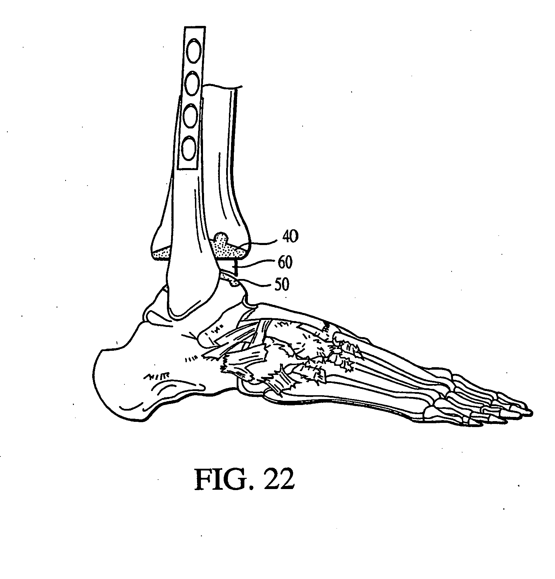

FIG. 22 is a similar view of the foot in FIG. 1 and showing the mobile bearing, ankle joint prosthesis components inserted, the ankle distraction removed and the fibula placed back in position and plated in place.

### DETAILED DESCRIPTION OF THE PREFERRED EMBODIMENT

Before explaining at least one embodiment of the present invention in detail, it is to be understood that the invention is not limited in its application to the details of construction and to the arrangements of the components set forth in the following description or illustrated in the drawings. The invention is capable of other embodiments and of being practiced and carried out in various ways. Also, it is to be understood that the phraseology and terminology employed herein are for the purpose of description and should not be regarded as limiting.

Referring now to the drawings wherein are shown preferred embodiments and wherein like reference numerals designate like elements throughout, there is shown in FIG. 1 a lateral, elevational view of a right, human foot that illustrates most of the foot bones and the lower ends of the lower leg bones. The present invention involves three of these bones: the fibula 2, the tibia 4 and the talus 6.

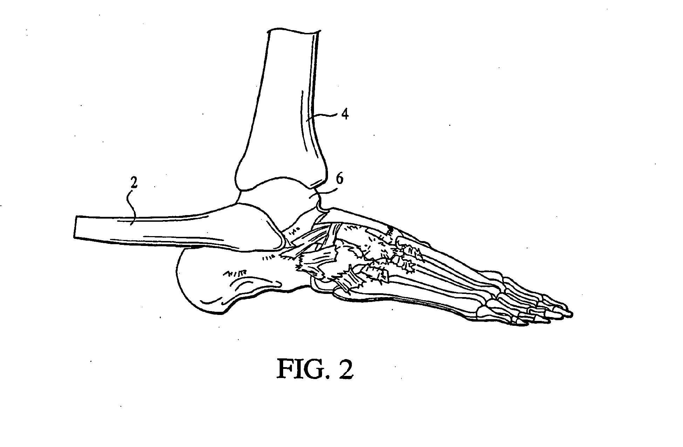

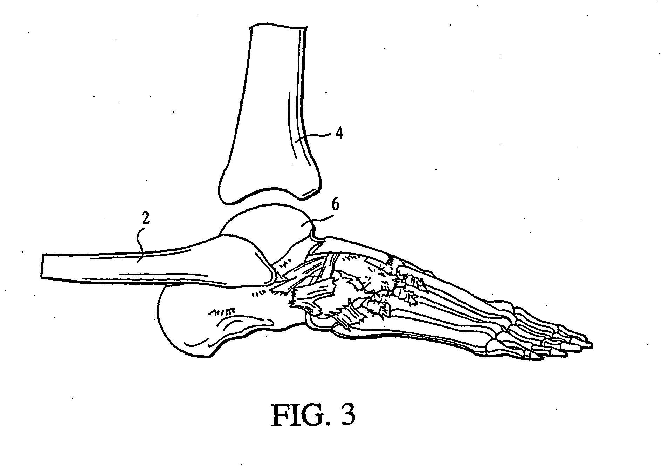

To gain lateral access to the ankle joint, the fibula 2 is cut at a point above its lower end and this lower end is moved to the side. See FIG. 2. The distal end of the fibula is reflected down hinged on the posterior talofibular and calcaneofibular ligaments. The syndesmosis, anterior talofibular and part of the calcaneofibular ligaments are released from the fibula. The ankle joint is distracted manually or by using an unilateral external fixator applied to the medial aspect. This gives better access to the distal tibia and talar dome surfaces that are to be cut. See FIG. 3.

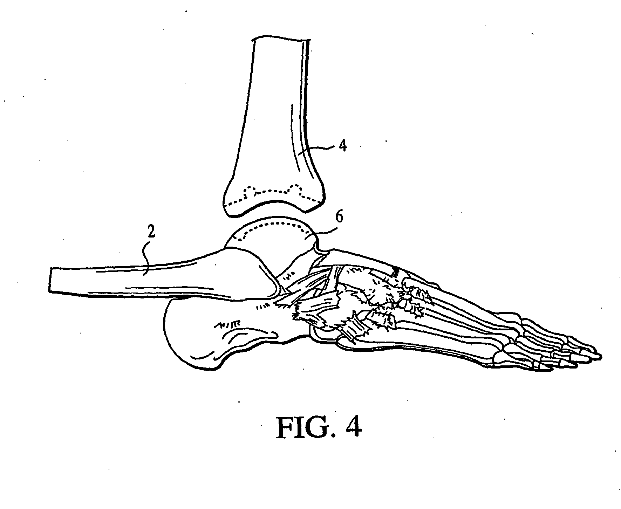

FIG. 4 shows by dashed lines the approximate shape of the cuts that are to be made in the surfaces of the distal tibia and talar dome. The geometry of these cuts is crescentic since it closely follows the natural anatomic contour of the joint and its subchondral bone of the distal tibia and talus.

The crescentic cut yields more bony surface area for cement fixation or bony ingrowth. The lateral approach, which exposes the ankle joint surface, allows a reproducible method for minimizing bone loss while performing accurate crescentic cuts. The precision of crescentic cuts that follows the natural anatomic contour of the joint and its subchondral bone, allows for the preservation of the strongest portions of the distal tibia and talus. This is the best bone in which to implant an ankle arthroplasty and may improve survival of ankle joints. An approximately flat cut may be used for the talus in instances of bony deformity which prevent the use of the crescentic saw. Furthermore the tenuous blood supply of the talus, which more typically is compromised with an anterior approach, is left intact with the lateral approach. The distal tibia blood supply is similarly preserved through a lateral approach. The lateral approach minimizes soft tissue compromise as opposed to the anterior approach.

Three basic cuts are required to remove the bone necessary to allow for implantation of the prosthesis: One crescentic, approximately flat, cut at the talar dome to remove diseased/damaged cartilage and bone. A second cut in the anterior-posterior direction to free the lateral aspect of the medial wall. A third and final, crescentic cut on the distal end of the tibia in a lateral-medial direction. This third cut will intersect with the second cut on the medial wall of the distal tibia to free the distal segment of tibial bone

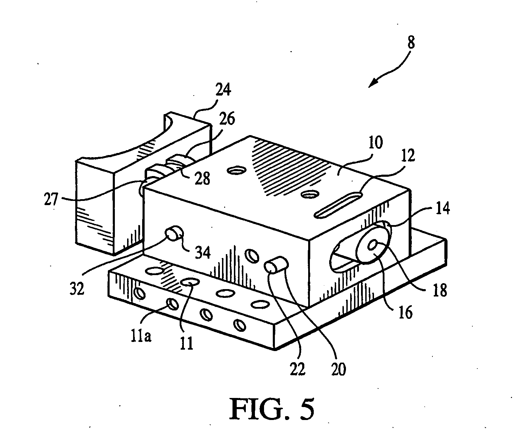

These cuts are made with the aid of a special cutting guide **8** that is shown in FIG. 5. It consists of a base **10** which has a plurality of anchoring holes **11** through which one or more anchoring or mounting pins may be extended for anchoring with the patient's tibia. Set screw holes **11a** in the side of the base allow set screws to be used to fix the position of the base relative to the pins that extend from the patient's tibia. The slotted hole **12** on the top of the base is placed over a mounting pin in the patient's tibia to provide a preferred initial means for securing the base to the patient's tibia.

At the back of the base, a slotted cavity **14** has been provided to allow for the placement of a locking cylinder **16** in the base **10.** The locking cylinder fits within this cavity with its distal end generally extending toward the front of the base. This cylinder has a hole that extends from its exterior surface and intersects a threaded bore **18** that extends along the axis of the cylinder from the cylinder's proximate end which is exposed in the entrance to base's rear cavity. The slotted hole **12** on the top of the base extends into the cavity **14** created in the rear of the base and then through the bottom portion of the base, so that this slotted hole **12** extends all the way through the base.

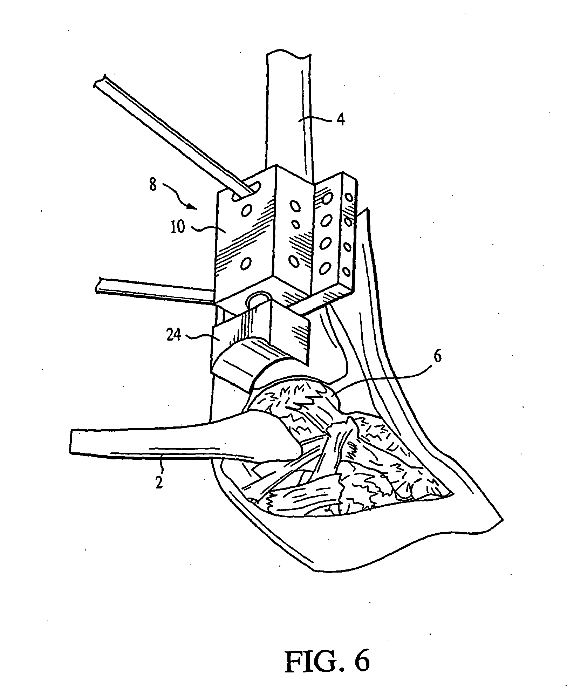

In use, see FIG. 6, the cylinder **16** is placed such that the pin from the patient's tibia also extends through the hole in the cylinder's exterior surface. The cylinder is secured to the pin by a set screw that fits within the cylinder's threaded, axial bore **18** and can be turned by accessing the set screw's free end which is exposed at the cylinder's proximate end. From each side of the base extend additional threaded bores **20** that project into the sides of the bases' rear cavity **14.** These bores contain set screws **22** that can be tighten to lock the cylinder **16** in place relative to the rest of the base **10.** The advantage of this configuration is that it provides the surgeon with a means to adjust the location of the base **10** relative to the locking cylinder **16** which is locked to the pin in the patient's tibia. Thus, the locking cylinder **16** can be moved to either side or rotated about a vertical axis defined by the axis of the pin that extends from the patient's tibia.

The centerlines of the anchoring holes **11** which are located in the base's top surface and towards its outer edges are slanted towards and at an angle with respect to the base's longitudinal centerline. This angle is provided so as to allow the pins that pass through these holes to approximately contact the tibia perpendicularly to its surface. This allows these pins to be directed towards the tibia's centerline so as to provide the pins with the most secure attachment to the bone.

To the front of this base **10** is attached a shelf **24;** the front surface of which is shaped so as to have the curvature that is desired to be used in the cut that is made in the surfaces of the distal tibia and talar dome. The surgeon places his curved blade against the shelf's front surface to guide his cutting during the surgical procedure.

The shelf **24** is mounted so that it can be pivoted up and down about a pivot joint **26** which is mounted on the back of the shelf. A set screw **27** allows the angular rotation of the pivot joint **26** to be fixed. Additionally, the nature of this connection is such that the shelf can easily be detached from the shaft. This allows for the opportunity to interchange the shelf that is attached to a shaft so that a shelf can be selected from a group of the various surface curvatures, with appropriate protrusions, that will be needed during the surgery.

From the shelf's pivot joint, there extends a shaft **28** which can slip into and out of a cavity that exists in the front of the base. On the side of the base there exist two additional bores **32** in which are placed set screws **34** that can be screwed inward so as to contact the exterior surface of the shaft **28** that extends into the base's front cavity. Thus, the distance between the back of the shelf and the front of the base can be set by the use of these set screws to lock the shaft relative to the base. With this configuration, the shelf's position is adjustable in three directions: forward and backward as the shaft moves in and out of the base, rotationally about the axis of the shaft which can rotate within the base's front cavity, and rotationally (i.e., up and down, assuming that the ends of the shelf are extending horizontally) about the hinge joint that connects the front of the shaft and the back of the shelf.

After the necessary incisions have been made to expose the ankle joint and the fibula has been cut and peeled back to allow full access to the joint, the cutting guide **8** is placed on the tibia at the location where its shelf **24** can best be utilized in making the necessary cuts on the talus and the tibia. In order to assist in achieving the ideal alignment of the bone cuts and prosthetic components, a modification of the cutting guide **8** may be introduced to facilitate restoration of anatomic, physiologic, and biomechanical alignment.

Pre-operative x-rays of the normal contra-lateral ankle are obtained. The normal ankle x-rays are compared to the diseased ankle. The side of the diseased ankle joint, which is the least damaged, is identified in both the anterior-to-posterior and lateral ankle x-rays. These points in the x-rays help to restore the normal architecture and ankle joint level.

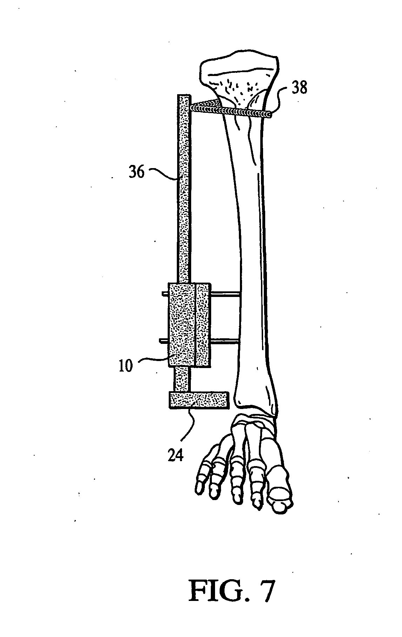

An alignment rod **36** is attached to the cutting guide **8** and extends proximally to the knee. See FIG. 7. The alignment rod **36** is held in this position and stabilized to the knee proximally with a coiled spring **38,** and to the ankle distally with the transfixion pins.

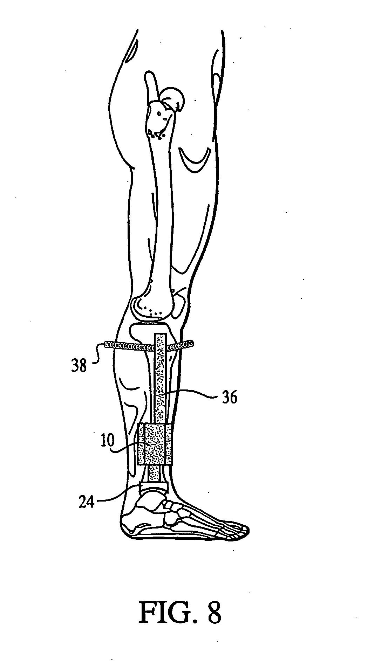

A lateral x-ray of the tibia is obtained and the alignment rod **36** is positioned to align centrally down the intramedually canal of the tibia from the knee, proximally, and the ankle, distally. See FIG. 8.

The alignment rod **36** is used as a reference for the alignment of the ankle joint line in the anterior to posterior plane, which is perpendicular to the alignment rod **36**.

To establish the alignment of the ankle joint in the lateral and medial plane, an anterior-to-posterior ankle x-ray is taken. A narrow wire is passed in the cutting guide **8** from lateral to medial on the anterior aspect of the ankle joint. The height of the crescentic cut is adjusted until the narrow wire is located just proximal to the portion of the ankle joint which has the least amount of damage as determined on the x-rays of the normal ankle joint.

The orientation above the tibia of the base's top, slotted hole **12** serves to define the site for the drilling of a hole for the insertion of a primary mounting pin in the tibia. After this pin is inserted, its extended end is fed through the base's top, slotted hole **12** and through the locking cylinder's hole. The cylinder's set screws are then tightened to be to lock the cutting guide **8** in place.

The surgeon next chooses one or more of the side holes **11** for use in anchoring to secondary mounting pins which are passed through these holes **11** and into the tibia. For each of these pins, a pilot hole is initially drilled in the bone and a secondary mounting pin is inserted through the hole **11.** A set screw **11a** is again used to lock the cutting guide to each of these secondary mounting pin. Once the cutting guide is securely locked to the mounting pins, the shelf **24** of the apparatus can be further adjusted and aligned to ensure that it is properly located to most effectively assist the surgeon in making the necessary cuts.

The cutting guide **8** may be made of any appropriate material, such as stainless steel, or the like, which is suitable for use in a surgical environment and is capable of being sterilized.

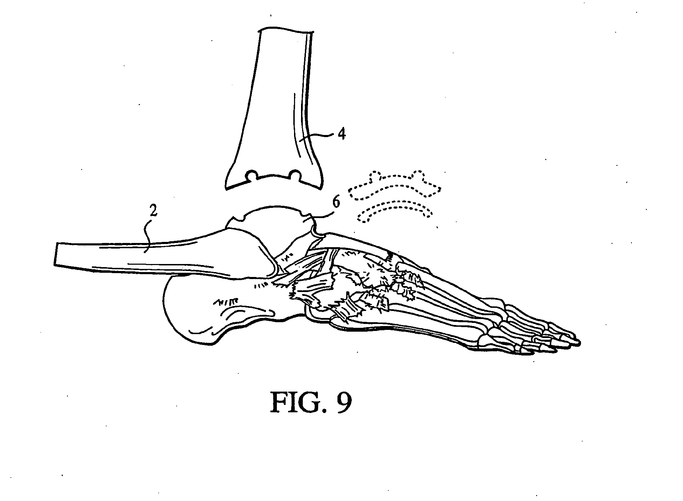

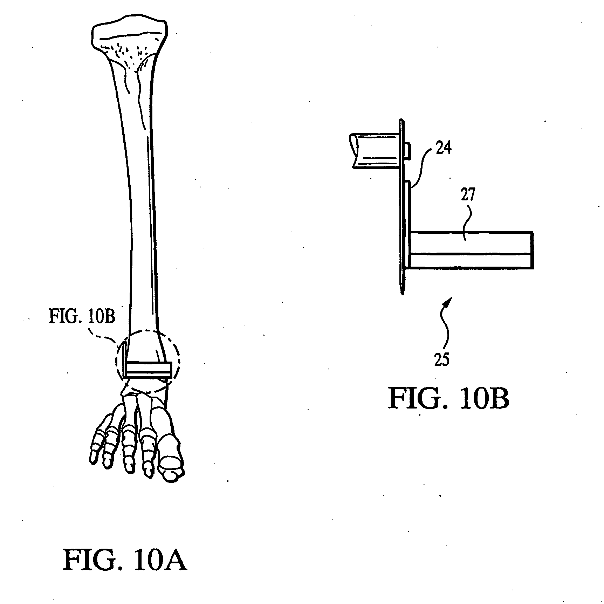

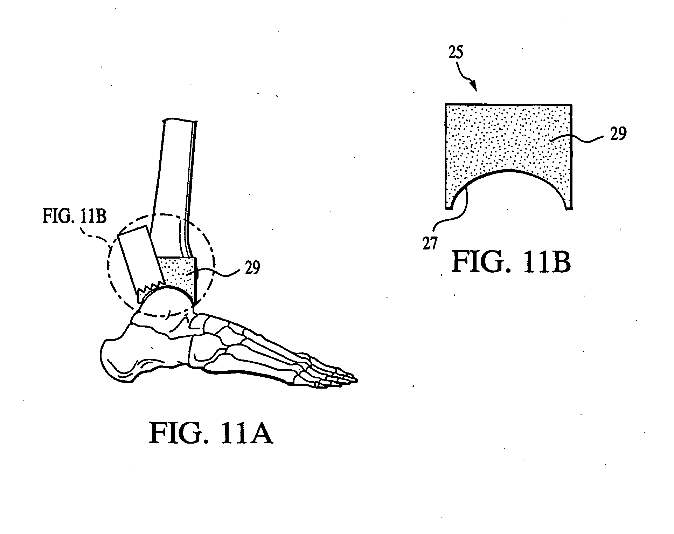

FIG. 9 shows the distal tibia and talar dome after the necessary portions have been removed. These portions are removed with the assistance of a talar, lateral surface cutting guide **25.** The talar lateral surface cutting guide is used to cut the lateral surface of the talus. This provides a flat mating surface for the inside of the talar component. This surface cutting guide **25** is composed of a curved piece **27** which acts as a cutting guide shelf and is adjoined to a vertical element **29** extending superior to inferior on the lateral edge of the cutting shelf. See FIGS. 10A and 10B. The curved piece **27** is narrow so that it can be positioned between the distracted tibia and talus. The curved piece **27** is then held flush against the superior aspect of the talus and pushed medially such that the lateral edge of the talus protrudes lateral to the vertical element of the cutting shelf. An oscillating saw is then used to cut the lateral aspect of the talus. This talar lateral surface cut is not required when using an approximately flat cut on the talus. See FIGS. 11A and 11B. These prepared surfaces correspond to the bone-metal interface surfaces of the prosthetic components.

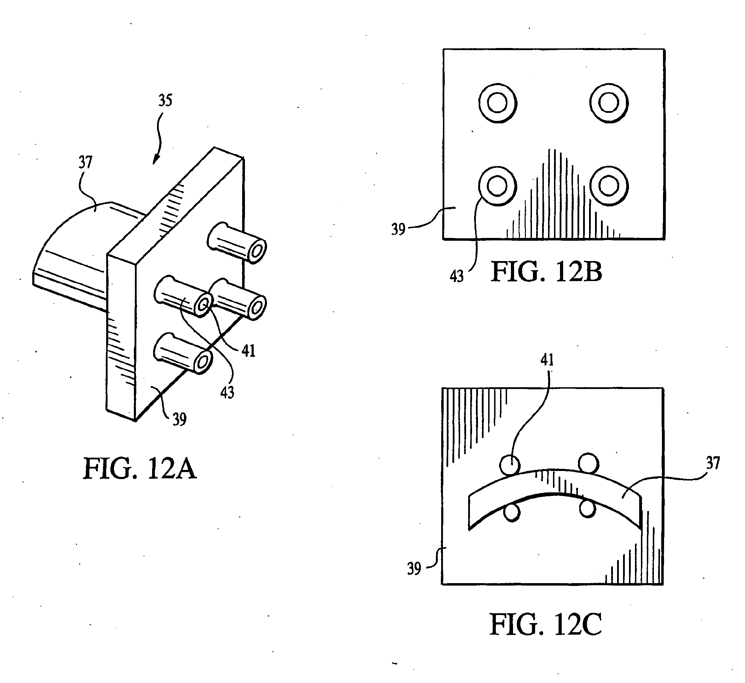

It can be seen that these prepared surfaces have one or more recesses that run from the lateral to the medial edges of the bones. These recesses are configured so as to match with comparably shaped protrusions and/or shoulders that are parts of the prosthesis' components. The protrusions run from lateral to medial on the prosthesis and allow for greater stability to ankle dorsiflexion and plantarflexion, which occur in a plane perpendicular to the protrusions of the prosthesis. These recesses are positioned and created with the assistance of a recess cutting guide **35.** See FIGS. 12A and 12B.

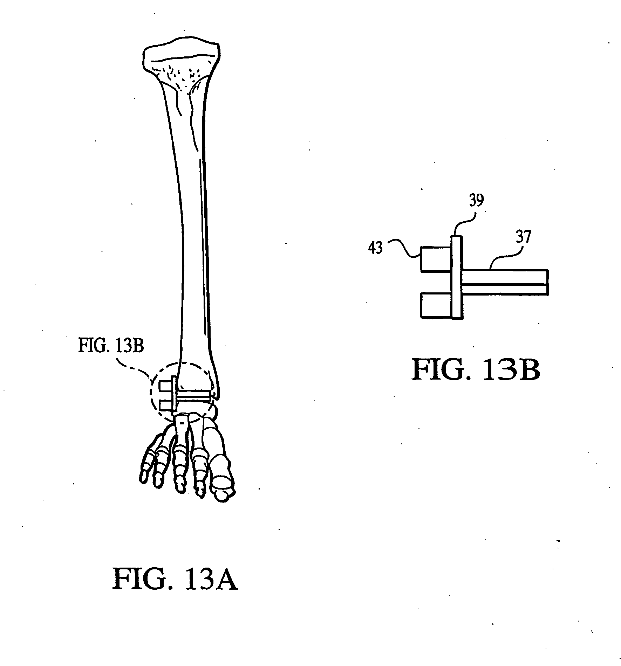

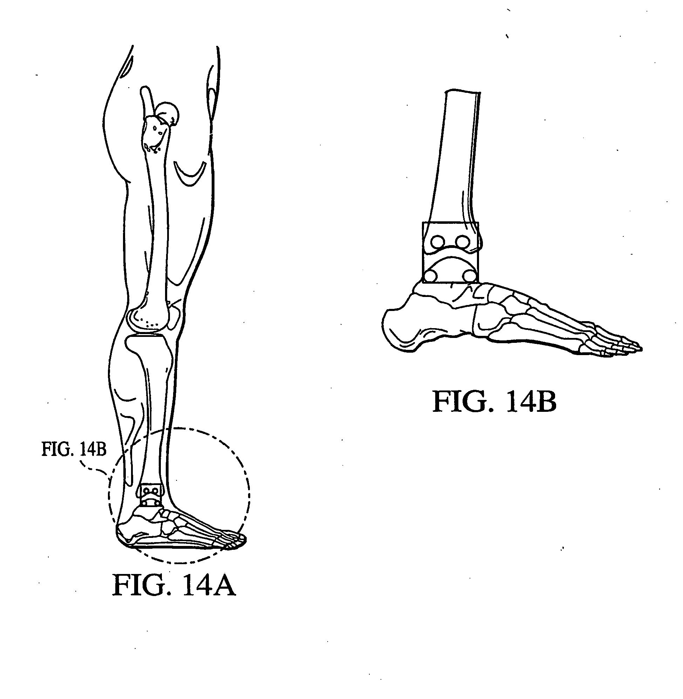

This recess cutting guide **35** has a curved shelf **37** that is attached to a vertical element **39** that runs both superiorly and inferiorly. In the vertical element are several holes **41** that have alignment tubes **43** extending from them. These holes and their alignment tubes extend parallel to the surface of curved shelf **37.** The curved shelf **37** is narrow so that it can be positioned between the distracted tibia and talus. See FIGS.13A and 13B. With the curved shelf **37** held firmly opposed to the superior surface of the talus, it is pinned to the talus with narrow wires through the alignment tubes positioned near the anterior and posterior aspects of the guide. A narrow wire is passed down the curved shelf 37 for each recess and the orientation and location of each narrow wire is inspected on anterior-to-posterior and lateral x-ray views of the ankle. Once the location of the recesses is determined, one or more stabilization recesses are created in the talus with a tapered drill, which tapers from lateral to medial. See FIGS. 14A and 14B.

The ankle joint prosthesis of the present invention can take the form of either a two or a three component embodiment, which may be referred to below as either the mobile bearing or the semi-constrained versions, respectively. These are separately described below.

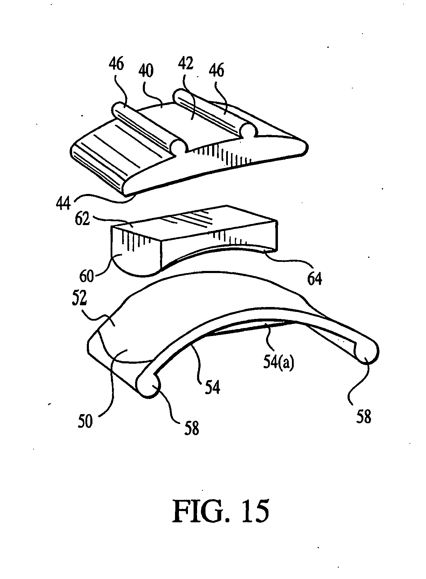

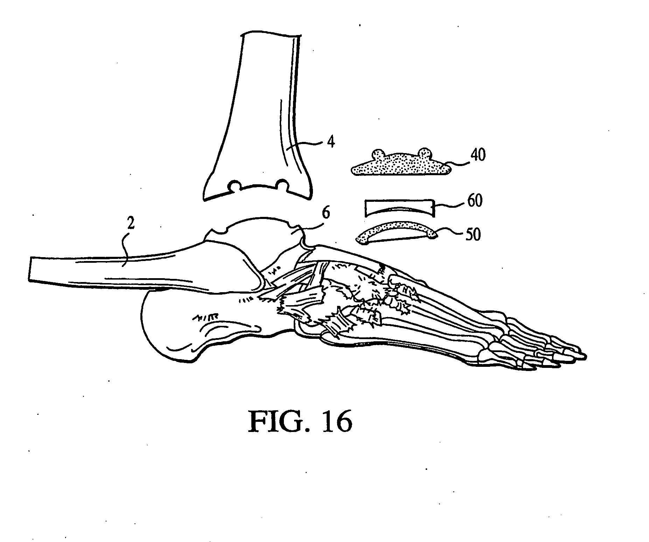

FIG. 15 is a perspective, side view of a preferred, mobile bearing embodiment of the ankle joint prosthesis of the present invention. It consists of tibial **40,** talar **50** and mobile bearing **60** components, where the tibial **40** and talar **50** components are mounted respectively on the prepared, crescentic-shaped tibial and talar surfaces. The mobile bearing component **60** is located between the tibial **40** and talar 50 components. See also FIG. 16.

The tibial component **40** has a top surface **42** that has convex curvature in the anterior to posterior direction so as to approximate the curvature of the prepared distal tibia surface. Its bottom surface **44** is flat. The top surface **42** has one or more lateral-to-medially aligned protruding surfaces **46** that are configured to match with the similarly shaped recesses that have been made in the tibia's prepared surface. These protrusions **46** serve to stabilize any motion of the component **40** relative to the prepared distal tibia surface and provide greater surface area for bony ingrowth or cement fixation of the component to the tibia. These protrusions may be tapered, from narrow medial to wide lateral, so as to create a more secure and stable fit as the device is inserted from lateral to medial. The component's top surface **42** may be coated with a substance to enhance bony ingrowth or cement fixation. This material may be sintered beads, plasma sprayed, implex/trabecular metal (implex) or other material that provides an interlocking mechanism.

The talar component **50** is a crescentic-shaped structure that is curved on both its top **52** and bottom **54** surfaces. Its the top surface **52** has convex curvature in the anterior to posterior direction and concave curvature in its lateral to medial direction. Its bottom surface **54** has concave curvature in the anterior to posterior direction that approximates the natural curvature found on the prepared talus dome. At some point on the talar component's bottom surface, preferably at its anterior and posterior edges, are protrusions or ridges **58** that extend downward from its bottom surface **54.** The shape of these protrusions **58** is configured to match with the similarly shaped recesses or extrusions that have been made in the talar dome's prepared surface. These protrusions **58** serve to stabilize any motion of this component **50** relative to the prepared talar dome surface and provide greater surface area for bony ingrowth or cement fixation of the component 50 to the talar dome.

The component's bottom surface **54** maybe coated with a substance to enhance bony ingrowth or cement fixation. The medial side of talar component 50 has a shoulder **54(a)** extending downward from the top surface that coincides with the prepared medial surface of the talus.

The talar component of the prosthesis effectively serves to semi-resurface the medial and lateral oblique joint surfaces (the "gutters") of the ankle. It does this by resurfacing the medial facet on the tibial side and the lateral facet on the talar side. Both the tibial and the talar portions of the superior articulating surface are fully resurfaced.

The mobile bearing component **60** has a flat top surface **62** and a saddle-shaped bottom surface **64.** The bottom surface **64** is configured with a saddle-shaped configuration so as to match with the talar component's top surface **52** and to allow for internal and external rotation motions. This saddle-shaped surface also allows for dorsiflexion and plantar flexion motion. The mobile bearing's top surface **62** is flat so as to match with the shape of the tibia component's flat, bottom surface **44.** This flat surface allows for internal and external rotation motions.

These components **40,50, 60** may preferably be made of any appropriate material suitable for the surgical environment. High density, ultra-high molecular weight polyethylene is an excellent plastic material for the bearing surfaces. It is widely used in other surgical devices and characterized by excellent wear resistance and a low coefficient of friction. Titanium or cobalt chrome alloys, or ceramics, are materials commonly used for the components that are rigidly attached to the bony surfaces.

The mobile bearing embodiment of ankle joint prosthesis described above can also have various versions. For example, in some application it is preferred to use a talar component **50** having a top surface **52** that has only convex curvature in the anterior to posterior direction. Since the bottom surface **64** of the mobile bearing component **60** must have matching and complimentary curvature, this surface **64** also has only convex curvature in the anterior to posterior direction. See FIGS. 17A-1, 17A-2, 17B-1 and 17B-2 for a comparison of the various types of curvatures that can be used in the mobile bearing component's bottom surface.

Due to talar bony deformities, it may also be useful to have a talar component that does not use the crescentic cut, but a flat cut For example, the talar dome may be depressed or collapsed and there is not room to perform the crescentic cut. A talar component **70,** again with various bearing surface curvatures **71** and **72,** may have a flat surface **73** on the inferior or bottom surface. Again a medial to lateral directed protrusion **74** provides great surface area for fixation and additional stability. See FIGS. 20C-1 through 20C-7 for possible talar component configurations with a flat cut on the bottom surface.

In a preferred, semi-constrained bearing embodiment, the prosthesis consists of a tibial **41,** semi-constrained bearing **61** and talar **51** components, where the tibial **41** and talar **51** components are mounted respectively on the prepared, crescentic-shaped tibial and talar surfaces. See FIG. 19A.

In this embodiment, the tibial component **41** has a top surface **43** that has convex curvature in the anterior to posterior direction so as to approximate the curvature of the prepared distal tibia surface. This top surface **43** has one or more lateral-to-medially aligned protruding surfaces **47** that are configured to match with the similarly shaped recesses that have been made in the tibia's prepared surface. These protrusions **47** serve to stabilize any motion of the component **41** relative to the prepared distal tibia surface and provide greater surface area for bony ingrowth or cement fixation of the component to the tibia. These protrusions **47** may be tapered, from narrow medial to wide lateral, so as to create a more secure and stable fit as the device is inserted from lateral to medial. The component's top surface **43** may be coated with a substance to enhance bony ingrowth or cement fixation. This material may be sintered beads, plasma sprayed, implex/trabecular metal (implex) or other material that provides an interlocking mechanism.

The tibial component's bottom surface **45** has one of a variety of forms of curvature that are designed to yield various degrees of constraint for the underlying semi-constrained bearing component **61.** This bearing component **61** is preferably made from polyethylene or other suitable bearing material, whereas, the tibial and talar components are made from one of a variety of suitable metals. See FIGS. 18A-1, 18A-2, 18B-1 and 18B-2 for a comparison of the various types of curvatures that can be used in the tibial component's bottom surface.

The semi-constrained bearing component's top surface may be bonded or mechanically attached to the bottom surface of the tibial component **41.** The semi-constrained bearing component **61** may also be partially locked into the tibial component **41.** For example, its superior aspect **63** may be slightly convex to match a slightly concave curvature that is placed in the bottom surface of the tibial component **41.** See FIG. 19A which shows a semi-constrained bearing embodiment in which the bearing component's top surface has a dome that partially locks this component into the matching concavity or recess **49** that exists in the tibial component's bottom surface. See FIG. **19B** for the bottom view of the tibial component shown in FIG. 19A. In locked and unlocked versions, the inferior aspect **65** of the semi-constrained bearing component **61** will have different configurations depending on the constraint required.

In this semi-constrained bearing embodiment, the talar component **51** has a top surface **53** with curvature that matches and is complementary to the curvature found in the semi-constrained bearing component's bottom surface **65.** The talar component's bottom **55** surface has concave curvature or is approximately flat in the anterior to posterior direction that approximates the natural curvature or flat saw cut found on the prepared talus dome. At some point on the talar component's bottom surface, preferably at its anterior and posterior edges, are protrusions or ridges **59** that extend downward from its bottom surface **57.** See FIG. 20A-1 to 20A-3 and 20B-1 to 20B-3. The shape of these protrusions **59** is configured to match with the similarly shaped recesses or extrusions that have been made in the talar dome's prepared surface. These protrusions **59** serve to stabilize any motion of this component **51** relative to the prepared talar dome surface and provide greater surface area for bony ingrowth of the component **51** to the talar dome. The lateral side of talar component **51** has a shoulder **55(a)** extending downward from the top surface that coincides with the prepared lateral surface of the talus. The component's bottom surface **55** may be coated with a substance to enhance bony ingrowth or cement fixation.

In the semi-constrained bearing embodiment, the tibial and talar components may be wider medially than laterally so as to approximated the native truncated cone shape of the talus.

To mount the tibial **40** and talar **50** components in place, the protrusions **46** and/or shoulders **56** of these component are carefully aligned with the matching recesses or groves that have been prepared in the respective distal tibia and talar dome surfaces. The medial edges of these components are abutted against the lateral edges of the grooves. A surgical mallet may be used to apply light blows to the lateral edges of the components to drive the medial edges of their protrusions and/or shoulders into the matching grooves until the components are fully seated in the respective tibial and talar dome surfaces. These components become adhered to tibial and talar dome cut surfaces by cement fixation or by press fitting and later bony ingrowth.

The mobile bearing component **60** may be properly located and seated within the ankle joint, between the tibial component **40** and the talar component **50,** by hand. The mobile bearing component is inserted into the ankle joint with the foot in distracted position. FIG. 21 illustrates the completed ankle joint for the mobile bearing embodiment of the prosthesis with all of the prosthesis' components in place. FIG. 22 illustrates the ankle prosthesis components inserted, the ankle distraction removed and the fibula placed back in position and plated in place.

If the completed ankle joint is too lax, a mobile bearing component 60 of greater thickness may be chosen. Similarly, if the completed ankle joint is too tight, a mobile bearing **60** of lesser thickness can be used. Selection of the mobile bearing component **60** of proper thickness permits adjustment of the overall height of the prosthesis.

For the semi-constrained bearing embodiment of the prosthesis, different thickness of the tibial or talar components are used to accomplish the same fitting objectives discussed above.

Although the foregoing disclosure relates to preferred embodiments of the invention, it is understood that these details have been given for the purposes of clarification only. Various changes and modifications of the invention will be apparent, to one having ordinary skill in the art, without departing from the scope of the invention as hereinafter set forth in the claims.

## Claims

1. An ankle joint prosthesis adapted to involve the patient's distal tibia and talus, said prosthesis comprising:
a tibial component (40) for lateral to medial implanting on a prepared portion of said distal tibia, said tibial component having a top (42) and a bottom (44) surface, said bottom surface being approximately flat,
a talar component (50) for lateral to medial implanting on a prepared portion of said talus, said talar component having a top (52) and a bottom (54) surface, said top surface (52) having convex curvature in the anterior to posterior direction, said bottom surface (54) having concave curvature in the anterior to posterior direction and configured so as to compliment and match with the curvature of said prepared portion of said talus, and
a mobile bearing component (60) for lateral to medial implanting between said tibial (40) and talar components (50), said bearing component having a top (62) and a bottom (64) surface, said top surface (62) being approximately flat, said bottom surface (64) having concave curvature in the anterior to posterior direction that is complementary to the curvature of said talar component's top surface (52) **characterised in that** said which component top surface (42) has a convex curvature in the anterior to posterior direction and is configured so as to compliment and match with the curvature of said prepared portion of said distal tibia.

2. The ankle joint prosthesis as recited in claim 1, wherein:
said tibial component top surface (42) having a lateral-to-medially aligned protrusion (46) that is configured to match with a similarly shaped recess that has been made in the prepared portion of said distal tibia.

3. The ankle joint prosthesis as recited in claim 1 or claim 2, wherein:
said talar component bottom surface (54) having a lateral-to-medially aligned protrusion (58) that extends downward from an edge chosen from the group of anterior and posterior edges, said protrusion (58) configured to match with a similarly shaped recess that has been made in the prepared portion of said talus.

4. The ankle joint prosthesis as recited in claim 1, 2 or 3, wherein:
said talar component having an anterior-to-posterior aligned shoulder (54(a)) that extends downward from the lateral edge of said talar component top surface.

5. The ankle joint prosthesis as recited in any one of claims 1-4, wherein:
said talar component top surface (52) having concave curvature in the lateral to medial direction, and
said mobile bearing component bottom surface (64) having convex curvature in the lateral to medial direction that is complementary to the curvature of said talar component's top surface (52) in the lateral to medial direction.

6. The ankle joint prosthesis as recited in claim 2, 3 or 4 wherein:
said tibial protrusion (46) being tapered, from narrow medial to wide lateral, so as to create a more secure and stable fit for said tibial component.

7. The ankle joint prosthesis as recited in any one of the preceding claims, wherein:
said tibial component top surface (42) being coated with a substance to enhance adhesion between said component and prepared, distal tibia surface.

8. The ankle joint prosthesis as recited in any one of the preceding claims, wherein:
said talar component bottom surface (54) being coated with a substance to enhance adhesion between said component and prepared, talar surface.

9. The ankle joint prosthesis as recited in claim 7 or 8, wherein:
said coating substance being chosen from the group consisting of sintered beads, plasma sprayed, implex/trabecular metal or other material that provides an interlocking mechanism for better adhesion between said tibial component (40) and prepared, distal tibia surface and/or between said talar component (50) and prepared, talar surface.

10. The ankle joint prosthesis as recited in any one of the preceding claims, wherein:
said components (40, 50, 60) being fabricated from materials chosen from the group consisting of an ultra-high, molecular weight polyethylene, titanium or cobalt chrome alloys and ceramic materials.

11. An ankle joint prosthesis adapted to involve the patient's distal tibia and talus, said prosthesis comprising:
a tibial component (41) for lateral to medial implanting on a prepared portion of said distal tibia, said tibial component having a top (43) and a bottom (45) surface,
a talar component (51) for lateral to medial implanting on a prepared portion of said talus, said talar component having a top (53) and a bottom (55) surface, said top surface (53) having convex curvature in the anterior to posterior direction, said bottom surface (55) having concave curvature in the anterior to posterior direction that is configured so as to compliment and match with the curvature of said prepared portion of said talus, and
a semi-constrained bearing component (61) for lateral to medial implanting between said tibial (41) and talar (51) components, said semi-constrained bearing component having a top (63) and a bottom (65) surface, said bottom surface (65) having concave curvature in the anterior to posterior direction that is complimentary to the curvature of said talar component's top surface, **characterised in that** said tibial component top surface (43) has a convex curvature in the anterior to posterior direction, and **in that**
said semi-constrained bearing component top surface (63) and said tibial component bottom surface (45) have complimentary curvature that is configured to aid in providing a defined level of constraint of movement and interaction between said semi-constrained bearing component top surface (63) and said tibial component bottom surface (45).

12. The ankle joint prosthesis as recited in claim 11, wherein:
said tibial component bottom surface (45) having concave curvature in the anterior to posterior direction, and
said semi-constrained bearing component top surface (63) having complimentary convex curvature in the anterior to posterior direction.

13. The ankle joint prosthesis as recited in claim 11 or 12, wherein:
a portion of said tibial component bottom surface (45) having a recess (49), and
a portion of said semi-constrained bearing component top surface (63) having a convex protrusion that is configured and located on said surface so as to be complimentary to said recess (49) in said tibial component bottom surface (45) and so as to partially constrain the relative motion between said surfaces.

14. The ankle joint prosthesis as recited in claim 11, wherein:
said tibial component top surface (43) having a lateral-to-medially aligned protrusion (47) that is configured to match with a similarly shaped recess that has been made in the prepared portion of said distal tibia.

15. The ankle joint prosthesis as recited in claim 11 or 12, wherein:
said talar component bottom surface (55) having a lateral-to-medially aligned protrusion (59) that extends downward from an edge chosen from the group of anterior and posterior edges, said protrusion configured to match with a similarly shaped recess that has been made in the prepared portion of said talus.

16. The ankle joint prosthesis as recited in claim 11, 12 or 13, wherein:
said talar component (51) having an anterior-to-posterior aligned shoulder (55(a)) that extends downward from the lateral edge of said talar component top surface.

17. The ankle joint prosthesis as recited in any one of claims 11-14, wherein:
said talar component top surface (53) having concave curvature in the lateral to medial direction, and
said bearing component bottom surface (65) having convex curvature in the lateral to medial direction that is complementary to the curvature of said talar component's top surface (53) in the lateral to medial direction.

18. The ankle joint prosthesis as recited in claim 14 or 17, wherein:
said tibial protrusion (47) being tapered, from narrow medial to wide lateral, so as to create a more secure and stable fit for said tibial component.

19. The ankle joint prosthesis as recited in any one of claims 11-18, wherein:
said tibial component top surface (43) being coated with a substance to enhance adhesion between said component and prepared, distal tibia surface.

20. The ankle joint prosthesis as recited in any one of claims 11-19, wherein:
said talar component bottom surface (55) being coated with a substance to enhance adhesion between said component and prepared, talar surface.

21. The ankle joint prosthesis as recited in claim 19 or 20, wherein:
said coating substance being chosen from the group consisting of sintered beads, plasma sprayed, implex/trabecular metal or other material that provides an interlocking, mechanism for better adhesion between said tibial component and prepared, distal tibia surface and/or between said tibial component and prepared, talar surface.

22. The ankle joint prosthesis as recited in any one of claims 11-21, wherein:
said components (41,51,61) being fabricated from materials chosen from the group consisting of an ultra-high, molecular weight polyethylene, titanium or cobalt chrome alloys and ceramic materials.

## Patentansprüche

1. Sprunggelenkprothese, die zur Verbindung mit dem distalen Schienbein (Tibia) und dem Sprungbein (Talus) eines Patienten angepasst ist, und welche aufweist:
eine tibiale Komponente (40) zur lateralen bis zur medialen Implantation auf einen vorbereiteten Bereich der genannten distalen Tibia, wobei die genannte tibiale Komponente eine obere (42) und eine untere Oberfläche (44) aufweist und die genannte untere Oberfläche angenähert eben ist,
eine talare Komponente (50) zur lateralen bis zur medialen Implantation auf einen vorbereiteten Bereich des genannten Talus, wobei die genannte talare Komponente eine obere (52) und eine untere Oberfläche (54) aufweist, die genannte obere Oberfläche (52) von vorn nach hinten konvex gekrümmt ist und die genannte untere Oberfläche (54) von vorn nach hinten konkav gekrümmt ist, und wobei die Krümmungen an diejenige des vorbereiteten Bereichs des genannten Talus angepasst sind und diese ergänzen, und
eine bewegliche Tragkomponente (60) für eine laterale bis mediale Implantation zwischen der genannten tibialen (40) und talaren Komponente (50), wobei die genannte Tragkomponente eine obere (62) und eine untere Oberfläche (64) aufweist, die genannte obere Oberfläche (62) etwa eben ist und die genannte untere Oberfläche (64) eine konkave Krümmung von vorn nach hinten aufweist, die zur Krümmung der oberen Oberfläche (52) der genannten talaren Komponente komplementär ist,
**dadurch gekennzeichnet, dass** die genannte obere Oberfläche (42) der tibialen Komponente von vorn nach hinten konvex gekrümmt ist und so ausgebildet ist, dass sie an die Krümmung des genannten vorbereiteten Bereichs der genannten distalen Tibia angepasst ist und diese ergänzt.

2. Sprunggelenkprothese nach Anspruch 1, bei der die genannte obere Oberfläche (42) der tibialen Komponente einen von der Seite zur Mitte ausgerichteten Vorsprung (46) aufweist, der so ausgebildet ist, dass er in eine ähnlich geformte Ausnehmung passt, welche in den vorbereiteten Bereich der genannten distalen Tibia eingearbeitet worden ist.

3. Sprunggelenkprothese nach Anspruch 1 oder 2, bei der die genannte untere Oberfläche (54) der talaren Komponente einen seitlich zur Mitte ausgerichteten Vorsprung (58) aufweist, der sich von der vorderen oder hinteren Kante nach unten erstreckt, wobei der Vorsprung (58) so ausgebildet ist, dass er in eine ähnlich geformte Ausnehmung passt, welche in dem vorbereiteten Bereich des genannten Sprungbeins ausgearbeitet wurde.

4. Sprunggelenkprothese nach Anspruch 1, 2 oder 3, bei der die genannte talare Komponente eine von vorn nach hinten gerichtete Schulter (54(a)) aufweist, die sich vom seitlichen Rand der genannten oberen Oberfläche der talaren Komponente nach unten erstreckt.

5. Sprunggelenkprothese nach einem der Ansprüche 1 bis 4, bei der die genannte obere Oberfläche (52) der talaren Komponente eine von der Seite zur Mitte verlaufende konkave Krümmung besitzt und die genannte untere Oberfläche (64) der beweglichen Tragkomponente eine konvexe Krümmung von der Seite zur Mitte aufweist, die mit der Krümmung der genannten oberen Oberfläche (52) der talaren Komponente in Richtung von der Seite zur Mitte komplementär ist.

6. Sprunggelenkprothese nach Anspruch 2, 3 oder 4, bei welcher der genannte tibiale Vorsprung (46) verjüngt ist, und zwar enger in der Mitte und breiter an den Seiten ist, so dass die Verbindung mit der genannten tibialen Komponente sicherer und stabiler wird.

7. Sprunggelenkprothese nach einem der vorstehenden Ansprüche, bei der die genannte obere Oberfläche (42) der tibialen Komponente mit einer Substanz beschichtet ist, die ein Anhaften dieser Komponente an der vorbereiteten Oberfläche der distalen Tibia verbessert.

8. Sprunggelenkprothese nach einem der vorstehenden Ansprüche, bei der die genannte untere Oberfläche (54) der talaren Komponente mit einer Substanz beschichtet ist, welche ein Anhaften dieser Komponente an der vorbereiteten talaren Oberfläche verbessert.

9. Sprunggelenkprothese nach Anspruch 7 oder 8, bei der die genannte Beschichtungssubstanz aus der folgenden Gruppe ausgewählt ist: gesinterte Kügelchen, plasmagesprühtes Metal, ein "trabeculares" Metal von Implex oder andere Materialien, welche eine gegenseitige feste Verbindung zwischen der genannten tibialen Komponente (40) und der vorbereiteten Oberfläche der distalen Tibia und/oder zwischen der genannten talaren Komponente (50) und der vorbereiteten talaren Oberfläche schaffen.

10. Sprunggelenkprothese nach einem der vorstehenden Ansprüche, bei der die genannten Komponenten (40, 50, 60) aus Werkstoffen hergestellt sind, die aus den folgenden ausgewählt sind: Polyethylen mit ultrahohem Molekulargewicht, Titan-Chrom-Legierungen, Kobalt-Chrom-Legierungen und keramischen Materialien.

11. Sprunggelenkprothese, die zur Verbindung mit dem distalen Schienbein (Tibia) und dem Sprungbein (Talus) eines Patienten angepasst ist, und welche aufweist:
eine tibiale Komponente (41) zur lateralen bis zur medialen Implantation auf einen vorbereiteten Bereich der genannten distalen Tibia, wobei die genannte tibiale Komponente eine obere (43) und eine untere Oberfläche (45) aufweist,
eine talare Komponente (51) zur lateralen bis zur medialen Implantation auf einen vorbereiteten Bereich des genannten Talus, wobei die genannte talare Komponente eine obere (53) und eine untere Oberfläche (55) aufweist, die genannte obere Oberfläche (53) von vorn nach hinten konvex gekrümmt ist und die genannte untere Oberfläche (55) von vorn nach hinten konkav gekrümmt ist, und wobei die Krümmungen an diejenige des vorbereiteten Bereichs des genannten Talus angepasst sind und diese ergänzen, und
eine teilgekoppelte ("semi-constrained") Tragkomponente (61) zur lateralen bis medialen Implantation zwischen der genannten tibialen (41) und talaren (51) Komponente, wobei diese teilgekoppelte Tragkomponente eine obere (63) und eine untere (65) Oberfläche aufweist und die untere Oberfläche (65) eine von vorn nach hinten verlaufende konkave Krümmung zeigt, die zur Krümmung der oberen Oberfläche der genannten talaren Komponente komplementär ist,
**dadurch gekennzeichnet, dass** die genannte obere Oberfläche (43) der tibialen Komponente eine konvexe Krümmung von vorn nach hinten aufweist, und dass die genannte obere Oberfläche (63) der teilgekoppelten Tragkomponente und die genannte untere (45) Oberfläche der tibialen Komponente eine komplementäre Krümmung aufweisen, welche derart konfiguriert ist, dass ein definierter Grad einer Zwangsführung der Bewegung und eine Wechselwirkung zwischen der genannten oberen Oberfläche (63) der teilgekoppelten Tragkomponente und der genannten unteren (45) Oberfläche der tibialen Komponente erzielt wird.

12. Sprunggelenkprothese nach Anspruch 11, bei der die genannte untere Oberfläche (45) der tibialen Komponente eine von vorn nach hinten verlaufende konkave Krümmung aufweist und die genannte obere Fläche (63) der teilgekoppelten Tragkomponente eine von vorn nach hinten verlaufende komplementäre konvexe Krümmung besitzt.

13. Sprunggelenkprothese nach Anspruch 11 oder 12, bei der ein Teil der genannten unteren Fläche (45) der tibialen Komponente mit einer Ausnehmung (49) versehen ist und ein Teil der genannten oberen Fläche (63) der teilgekoppelten Tragkomponente einen konvexen Vorsprung aufweist, der auf der Fläche derart positioniert und ausgestaltet ist, dass er zur genannten Ausnehmung (49) in der genannten unteren Fläche (45) der tibialen Komponente komplementär ist und die Relativbewegung der beiden genannten Flächen teilweise einschränkt.

14. Sprunggelenkprothese nach Anspruch 11, bei der die genannte obere Oberfläche (43) der tibialen Komponente einen von der Seite zur Mitte ausgerichteten Vorsprung (47) aufweist, der so ausgebildet ist, dass er in eine ähnlich geformte Ausnehmung passt, welche in den vorbereiteten Bereich der genannten distalen Tibia eingearbeitet worden ist.

15. Sprunggelenkprothese nach Anspruch 11 oder 12, bei der die genannte untere Oberfläche (55) der talaren Komponente einen seitlich zur Mitte ausgerichteten Vorsprung (59) aufweist, der sich von der vorderen oder hinteren Kante nach unten erstreckt, wobei dieser Vorsprung so ausgebildet ist, dass er in eine ähnlich geformte Ausnehmung passt, welche in dem vorbereiteten Bereich des genannten Sprungbeins ausgearbeitet wurde.

16. Sprunggelenkprothese nach Anspruch 11, 12 oder 13, bei der die genannte talare Komponente (51) eine von vorn nach hinten ausgerichtete Schulter (55(a)) aufweist, die sich vom seitlichen Rand der genannten oberen Oberfläche der talaren Komponente nach unten erstreckt.

17. Sprunggelenkprothese nach einem der Ansprüche 11 bis 14, bei der die genannte obere Oberfläche (53) der talaren Komponente eine von der Seite zur Mitte verlaufende konkave Krümmung besitzt und die genannte untere Oberfläche (65) der Tragkomponente eine konvexe Krümmung von der Seite zur Mitte aufweist, die zur Krümmung der genannten oberen Oberfläche (53) der talaren Komponente in Richtung von der Seite zur Mitte komplementär ist.

18. Sprunggelenkprothese nach Anspruch 14 oder 17, bei welcher der genannte tibiale Vorsprung (47) verjüngt ist, und zwar enger in der Mitte und breiter an den Seiten ist, so dass die Verbindung mit der genannten tibialen Komponente sicherer und stabiler wird.

19. Sprunggelenkprothese nach einem der Ansprüche 11 bis 18, bei der die genannte obere Oberfläche (43) der tibialen Komponente mit einer Substanz beschichtet ist, die ein Anhaften dieser Komponente an der vorbereiteten Oberfläche der distalen Tibia verbessert.

20. Sprunggelenkprothese nach einem der Ansprüche 11 bis 19, bei der die genannte untere Oberfläche (55) der talaren Komponente mit einer Substanz beschichtet ist, welche ein Anhaften dieser Komponente an der vorbereiteten talaren Oberfläche verbessert.

21. Sprunggelenkprothese nach Anspruch 19 oder 20, bei der die genannte Beschichtungssubstanz aus der folgenden Gruppe ausgewählt ist: gesinterte Kügelchen, plasmagesprühtes Metal, ein "trabeculares" Metal von Implex oder andere Materialien, welche eine gegenseitige feste Verbindung zwischen der genannten tibialen Komponente und der vorbereiteten Oberfläche der distalen Tibia und/oder zwischen der genannten tibialen Komponente und der vorbereiteten talaren Oberfläche schaffen.

22. Sprunggelenkprothese nach einem der Ansprüche 11 bis 21, bei der die genannten Komponenten (41, 51, 61) aus Werkstoffen hergestellt sind, die aus den folgenden ausgewählt sind: Polyethylen mit ultrahohem Molekulargewicht, Titan-Chrom-Legierungen, Kobalt-Chrom-Legierungen und keramische Materialien.

## Revendications

1. Prothèse de l'articulation de la cheville, adaptée pour se combiner avec le tibia distal et le talus d'un patient, ladite prothèse comprenant:
un composant de tibia 40) pour l'implantation latérale à médiale sur une zone préparée dudit tibia distal, ledit composant de tibia comprenant une surface supérieure (42) et une surface inférieure (44), ladite surface inférieure étant approximativement plate,
un composant de talus (50) pour l'implantation latérale à médiale sur une zone préparée dudit talus, ledit composant de talus comportant une surface supérieure (52) et une surface inférieure (54), ladite surface supérieure (52) présentant une courbure convexe dans la direction antérieure à postérieure, ladite surface inférieure (54) présentant une courbure concave dans la direction antérieure à postérieure et étant configurée pour être complémentaire à la courbure de ladite zone préparée dudit talus et pour s'y adapter, et
un composant de support mobile (60) pour l'implantation latérale à médiale entre ledit composant de tibia (40) et le composant de talus (50), ledit composant de support comportant une surface supérieure (62) et une surface inférieure (64), ladite surface supérieure (62) étant approximativement plate et ladite surface inférieure (64) présentant une courbure concave dans la direction antérieure à postérieure complémentaire à la courbure de la surface supérieure (52) dudit composant de talus,
**caractérisée en ce que** ladite surface supérieure (42) dudit composant de tibia présente une courbure convexe dans la direction antérieure à postérieure et est configurée pour être complémentaire à la courbure de ladite zone préparée dudit tibia distal et pour s'y adapter.

2. Prothèse de l'articulation de la cheville selon la revendication 1, dans laquelle ladite surface supérieure (42) du composant de tibia présente une saillie (46) à alignement latéral à médial qui est configurée pour s'adapter dans un évidement de forme similaire pratiqué dans la zone préparée dudit tibia distal.

3. Prothèse de l'articulation de la cheville selon la revendication 1 ou 2, dans laquelle ladite surface inférieure (54) du composant de talus présente une saillie (58) à alignement latéral à médial qui s'étend vers le bas à partir d'un bord antérieur ou postérieur, ladite saillie (58) étant configurée pour s'adapter dans un évidement de forme similaire qui a été pratiqué dans la zone préparée dudit talus.

4. Prothèse de l'articulation de la cheville selon la revendication 1, 2 ou 3, dans laquelle ledit composant de talus présente un épaulement (54(a)) à alignement antérieur à postérieur qui s'étend vers le bas à partir du bord latéral de ladite surface supérieure du composant de talus.

5. Prothèse de l'articulation de la cheville selon l'une quelconque des revendications 1 à 4, dans laquelle ladite surface supérieure (52) du composant de talus présente une courbure concave dans la direction latérale à médiale, et que ladite surface inférieure (64) du composant de support mobile présente une courbure convexe dans la direction latérale à médiale qui est complémentaire à la courbure de ladite surface supérieure (52) du composant de talus dans la direction latérale à médiale.

6. Prothèse de l'articulation de la cheville selon la revendication 2, 3 ou 4, dans laquelle ladite saillie tibiale (46) est effilée, étant étroite au milieu et large sur les côtés, afin de créer un ajustage plus sûr et stable dudit composant de tibia.

7. Prothèse de l'articulation de la cheville selon l'une des revendications précédentes, dans laquelle ladite surface supérieure (42) du composant de tibia est revêtue d'une substance apte à augmenter l'adhésion entre ledit composant et la surface préparée du tibia distal.

8. Prothèse de l'articulation de la cheville selon l'une des revendications précédentes, dans laquelle ladite surface inférieure (54) du composant de talus est revêtue d'une substance apte à augmenter l'adhésion entre ledit composant et la surface préparée du talus.

9. Prothèse de l'articulation de la cheville selon la revendication 7 ou 8, dans laquelle ladite substance de revêtement est choisie parmi le groupe constitué des perles frittées, des dépôts métalliques produits par plasma, d'un métal trabéculaire (trabecular metal) d'implex ou d'un autre matériau qui fournit un mécanisme d'enchevêtrement pour une meilleure adhésion entre ledit composant de tibia (40) et la surface préparée du tibia distal et/ou entre ledit composant de talus (50) et la surface de talus préparée.

10. Prothèse de l'articulation de la cheville selon l'une des revendications précédentes, dans laquelle lesdits composants (40, 50, 60) sont fabriqués à partir de matériaux choisis dans le groupe formé par un polyéthylène à poids moléculaire extrêmement élevé, des alliages de titane ou de cobalt avec du chrome et les matériaux céramiques.

11. Prothèse de l'articulation de la cheville adaptée pour se combiner avec le tibia distal et le talus d'un patient, ladite prothèse comprenant:
un composant de tibia (41) pour l'implantation latérale à médiale sur une zone préparée dudit tibia distal, ledit composant de tibia comprenant une surface supérieure (43) et une surface inférieure (45),
un composant de talus (51) pour l'implantation latérale à médiale sur une zone préparée dudit talus, ledit composant de talus comportant une surface supérieure (53) et une surface inférieure (55), ladite surface supérieure (53) présentant une courbure convexe dans la direction antérieure à postérieure, ladite surface inférieure (55) présentant une courbure concave dans la direction antérieure à postérieure qui est configurée pour être complémentaire à la courbure de ladite zone préparée dudit talus et pour s'y adapter, et
un composant de support semi-contraint (61) pour l'implantation latérale à médiale entre ledit composant de tibia (41) et le composant de talus (51), ledit composant de support semi-contraint comportant une surface supérieure (63) et une surface inférieure (65), ladite surface inférieure (65) présentant une courbure concave dans la direction antérieure à postérieure qui est complémentaire à la courbure de ladite surface supérieure du composant de talus,
**caractérisée en ce que** ladite surface supérieure (43) du composant de tibia présente une courbure convexe dans la direction antérieure à postérieure, et **en ce que** ladite surface supérieure (63) du composant de support semi-contraint et ladite surface inférieure (45) dudit composant de tibia présentent une courbure complémentaire configurée pour aider à fournir un degré défini de contrainte de mouvement et d'interaction entre ladite surface supérieure (63) du composant de support semi-contraint et ladite surface inférieure (45) du composant de tibia.

12. Prothèse de l'articulation de la cheville selon la revendication 11, dans laquelle ladite surface inférieure (45) du composant de tibia présente une courbure concave dans la direction antérieure à postérieure, et ladite surface supérieure (63) dudit composant de support semi-contraint présente une courbure convexe complémentaire dans la direction antérieure à postérieure.

13. Prothèse de l'articulation de la cheville selon la revendication 11 ou 12, dans laquelle une partie de ladite surface inférieure (45) du composant de tibia comporte un évidement (49), et une partie de ladite surface supérieure (63) du composant de support semi-contraint présente une saillie convexe qui est configurée et située sur ladite surface pour être complémentaire audit évidement (49) de ladite surface inférieure (45) du composant de tibia et pour contraindre partiellement le mouvement relatif entre lesdites surfaces.

14. Prothèse de l'articulation de la cheville selon la revendication 11, dans laquelle ladite surface supérieure (43) du composant de tibia présente une saillie (47) à alignement latéral à médial qui est configurée pour s'adapter dans un évidement de forme similaire pratiqué dans la zone préparée dudit tibia distal.

15. Prothèse de l'articulation de la cheville selon la revendication 11 ou 12, dans laquelle ladite surface inférieure (55) du composant de talus présente une saillie (59) à alignement latéral à médial qui s'étend vers le bas à partir d'un bord antérieur ou postérieur, ladite saillie étant configurée pour s'adapter dans un évidement de forme similaire qui a été pratiqué dans la zone préparée dudit talus.

16. Prothèse de l'articulation de la cheville selon la revendication 11, 12 ou 13, dans laquelle ledit composant de talus (51) présente un épaulement (55(a)) à alignement antérieur à postérieur qui s'étend vers le bas à partir du bord latéral de ladite surface supérieure du composant de talus.

17. Prothèse de l'articulation de la cheville selon l'une quelconque des revendications 11 à 14, dans laquelle ladite surface supérieure (53) du composant de talus présente une courbure concave dans la direction latérale à médiale, et que ladite surface inférieure (65) du composant de support mobile présente une courbure convexe dans la direction latérale à médiale qui est complémentaire à la courbure de ladite surface supérieure (53) du composant de talus dans la direction latérale à médiale.

18. Prothèse de l'articulation de la cheville selon la revendication 14 ou 17, dans laquelle ladite saillie tibiale (47) est effilée, étant étroite au milieu et large sur les côtés, afin de créer un ajustage plus sûr et stable dudit composant de tibia.

19. Prothèse de l'articulation de la cheville selon l'une des revendications 11 à 18, dans laquelle ladite surface supérieure (43) du composant de tibia est revêtue d'une substance apte à augmenter l'adhésion entre ledit composant et la surface préparée du tibia distal.

20. Prothèse de l'articulation de la cheville selon l'une des revendications 11 à 19, dans laquelle ladite surface inférieure (55) du composant de talus est revêtue d'une substance apte à augmenter l'adhésion entre ledit composant et la surface préparée du talus.

21. Prothèse de l'articulation de la cheville selon la revendication 19 ou 20, dans laquelle ladite substance de revêtement est choisie parmi le groupe constitué des perles frittées, des dépôts métalliques produits par plasma, d'un métal trabéculaire (trabecular metal) d'implex ou d'un autre matériau qui fournit un mécanisme d'enchevêtrement pour une meilleure adhésion entre ledit composant de tibia et la surface préparée du tibia distal, et/ou entre ledit composant de talus et la surface préparée de talus.

22. Prothèse de l'articulation de la cheville selon l'une des revendications 11 à 21, dans laquelle lesdits composants (41, 51, 61) sont fabriqués à partir des matériaux choisis parmi ceux du groupe formé par un polyéthylène à poids moléculaire extrêmement élevé, les alliages de titane ou de cobalt avec du chrome, et les matériaux céramiques.
